# EUROPEAN PATENT APPLICATION

(11) **EP 4 714 472 A1**
(43) Date of publication of application: **25.03.2026**
(21) Application number: 24835398.9
(22) Date of filing: 04.07.2024
(51) Int. Cl.: A61L 24/10, A61L 24/06, A61L 24/00

(54) **TWO-COMPONENT IN-SITU ADHESIVE BASED ON SUPERCHARGED PROTEIN, AND PREPARATION METHOD THEREFOR AND USE THEREOF**

(30) Priority: 05.07.2023 CN 202310822652
(71) Applicant: Beijing Lanthanide Biotechnology Co., Ltd, Beijing 102629 (CN)
(72) Inventor: SUN, Yao, Beijing 102629 (CN); ZHANG, Nana, Beijing 102629 (CN); YU, Qiang, Beijing 102629 (CN)
(74) Representative: Murgitroyd & Company
(86) International application number: PCT/CN2024/103604
(87) International publication number: WO 2025/007918

(57) **Abstract**

The present application provides a two-component in-situ adhesive based on supercharged protein and preparation method and use thereof. The two-component in-situ adhesive of the present application may be used for in-situ and rapid gelatinization at tissue wounds, showing excellent adhesion strength between tissues, and also showing excellent adhesion performance even on the moist tissue surface; after the gelatinization, its adhesive surface is soft, and may be used to achieve the effects of moist wound hemostasis, wound restoration and the like, for example, it may be used for assisting hemostasis in vascular reconstruction closure, or assisting suture in cranial suture site; at the same time, the adhesive of the present application also has the advantages of simple and rapid preparation process, no need for special devices, rapid adhesion and biocompatibility and the like. It may be used as a new generation of a portable instantaneous biomedical adhesive, and it has great application prospects in situations such as acute bleeding of wounds and complex wound surface treatment.

## Description

### Cross-Reference to Related Application

The present application claims priority to a Chinese patent application with application number 202310822652.2 and invention title "Two-Component In-Situ Adhesive Based on Supercharged Protein, and Preparation Method and Use Thereof", filed on July 5, 2023, and the entire content of which is incorporated into this article by reference.

### Technical Field

The present application relates to the field of biomaterial technology, specifically to a two-component in-situ adhesive based on supercharged protein and preparation method and use thereof.

### Background of the Invention

Tissue adhesives refer to a type of biological materials that may interact with *in vivo* tissues, as to cause adhesion between tissues or between tissues and non-tissues (such as implants), and have functions such as controlling bleeding (stopping bleeding) and preventing gases and liquid from flowing (sealing). With the development of modern medicine, it is required that in the process of surgical operations, not only its function and appearance should be maximally restored, but also patients' pain should be minimized as much as possible. At present, in the operations for treatment of larger tissue defects or bleeding points, a suture or rivet mode is often used, it uses physical methods to pull and restore the tissues, but it does not achieve better results. The main reasons are as follows: sutures and rivets do not provide additional effects such as wound healing and anti-inflammatory besides simple physical pulling; the sutures and rivets may cause damage and bleeding to surrounding tissues in the process of the operations; after the wound healing, there may be scar hyperplasia and additional pain in removing the sutures. The use of the medical tissue adhesives may effectively solve these problems. In addition, rapid hemostasis and wound adhesion functions of the tissue adhesives are particularly suitable for treating injury states difficult to suture at the first time in wartime environments, and even for treating internal wounds that are difficult to handle with hemostatic bandages; in addition, the tissue adhesives may quickly seal wounds while also preventing further contact between the wounds and the external environment so as to achieve the effect of infection prevention. It is reported that a large proportion of battlefield deaths in the war occur during injury to arrival to field hospitals. If the efficient tissue adhesives containing hemostatic and analgesic drugs and the like may be used, more lives may be saved. In addition, with the development of clinical medical technologies, minimally invasive technologies are widely used already in various clinical departments. However, since wounds of minimally invasive operations are small, and the visual field and operability are limited, it is difficult to use the traditional mechanical fixation technologies for wound tissues mentioned above during operation. Therefore, the tissue adhesives have extremely broad application prospects in surgical operations, plastic surgeries, battlefield rescue, and other fields.

However, it is a great challenge for achieving ideal tissue interface adhesion, and there are two main reasons: on the one hand, there is a large amount of liquid present on the tissue surface, it presents a large amount of adhesives from breaking through a hydration layer to generate an effective intermolecular force with the tissue interface, thereby the adhesion performance of the adhesives is greatly reduced; and on the other hand, the soft properties of the tissues lead to its extremely high requirements for the compliance of the adhesives, namely the adhesives are required to be able to adaptively deform and adjust with the tissue activities, the biocompatibility is also a point that needs to be highly valued, and therefore, this has extremely high requirements for the adhesion properties and compositions of the adhesives, it greatly increases the difficulty of developing super strong tissue adhesives.

Based on the above research status, the research and development of instantaneous in-situ adhesives based on bioengineered proteins is of great significance for basic scientific research and biomedical applications.

### Summary of the Invention

### Purpose of the Invention

The purpose of the present application is to provide a two-component instantaneous in-situ adhesive based on bioengineered protein with excellent adhesive performance, and preparation method and use thereof.

### Solution Scheme

To achieve the above purpose, the present application provides the following technical schemes: In the first aspect, the present application provides a two-component in-situ adhesive based on a supercharged protein, comprising:
(1) an elastin-like protein rich in an amino functional group, and
(2) a compound containing a succinimide group.

As a preferred scheme, the elastin-like protein rich in the amino functional group contains n [(VPGKG)₉] repeating units, with each pair of [(VPGKG)₉] repeating units separated by a spacer (VPGXG); herein, n is an integer between 3~18, and X is any natural amino acids except proline; and/or, the compound containing the succinimide group is any one or more selected from the following: bis-succinimide succinate polyethylene glycol (SS-PEG-SS), succinimide acetate, and disulfide bis-succinimide propionate.

Further preferably, in the elastin-like protein rich in the amino functional group, the number n of repeating units [(VPGKG)₉] is an integer between 8~18, and/or, in the spacer (VPGXG), X is valine, arginine, leucine, isoleucine, or alanine; and/or, the compound containing the succinimide group is SS-PEG-SS.

In a specific implementation scheme, the elastin-like protein rich in the amino functional group contains an amino acid sequence selected from or composed of the following: SEQ ID NO: 1, SEQ ID NO: 2, SEQ ID NO: 3, SEQ ID NO: 4, and SEQ ID NO: 5; and/or, the compound containing the succinimide group is SS-PEG-SS, herein the average relative molecular weight of polyethylene glycol is between 2000-10000.

In a preferred specific implementation scheme, the elastin-like protein rich in the amino functional group contains an amino acid sequence selected from or composed of the following: SEQ ID NO: 3, SEQ ID NO: 4, and SEQ ID NO: 5; and/or, the compound containing the succinimide group is SS-PEG-SS, herein the average relative molecular weight of polyethylene glycol is between 5000-10000.
SEQ ID NO:1 
VPGKGVPGKGVPGKGVPGKGVPGKGVPGKGVPGKGVPGKGVPGKGVPGVGVPGK
GVPGKGVPGKGVPGKGVPGKGVPGKGVPGKGVPGKGVPGKGVPGVGVPGKGVPGKGV
PGKGVPGKGVPGKGVPGKGVPGKGVPGKGVPGKG; herein, the underlined part is repeating
 unit [(VPGKG)₉], and the bold-type part is the spacer (VPGXG);
SEQ ID NO:2 
VPGKGVPGKGVPGKGVPGKGVPGKGVPGKGVPGKGVPGKGVPGKGVPGVGVPGK
GVPGKGVPGKGVPGKGVPGKGVPGKGVPGKGVPGKGVPGKGVPGAGVPGKGVPGKGV
PGKGVPGKGVPGKGVPGKGVPGKGVPGKGVPGKGVPGVGVPGKGVPGKGVPGKGVPG
KGVPGKGVPGKGVPGKGVPGKGVPGKG; herein, the underlined part is repeating unit
 [(VPGKG)₉], and the bold-type part is the spacer (VPGXG);
SEQ ID NO:3 
VPGKGVPGKGVPGKGVPGKGVPGKGVPGKGVPGKGVPGKGVPGKGVPGVGVPGKGVP
GKGVPGKGVPGKGVPGKGVPGKGVPGKGVPGKGVPGKGVPGVGVPGKGVPGKGVPGK
GVPGKGVPGKGVPGKGVPGKGVPGKGVPGKGVPGVGVPGKGVPGKGVPGKGVPGKGV
PGKGVPGKGVPGKGVPGKGVPGKGVPGRGVPGKGVPGKGVPGKGVPGKGVPGKGVPG
KGVPGKGVPGKGVPGKGVPGVGVPGKGVPGKGVPGKGVPGKGVPGKGVPGKGVPGKG
VPGKGVPGKGVPGVGVPGKGVPGKGVPGKGVPGKGVPGKGVPGKGVPGKGVPGKGVP
GKGVPGVGVPGKGVPGKGVPGKGVPGKGVPGKGVPGKGVPGKGVPGKGVPGKG;
 herein, the underlined part is repeating unit [(VPGKG)₉], and the bold-type part is the spacer (VPGXG);
SEQ ID NO:4 
VPGKGVPGKGVPGKGVPGKGVPGKGVPGKGVPGKGVPGKGVPGKGVPGVGVPGKGVP
GKGVPGKGVPGKGVPGKGVPGKGVPGKGVPGKGVPGKGVPGVGVPGKGVPGKGVPGK
GVPGKGVPGKGVPGKGVPGKGVPGKGVPGKGVPGKGVPGAGVPGKGVPGKGVPGKGVPGKGV
PGKGVPGKGVPGKGVPGKGVPGKGVPGKGVPGVGVPGKGVPGKGVPGKGVPGKGVPGKGVPG
KGVPGKGVPGKGVPGKGVPGKGVPGVGVPGKGVPGKGVPGKGVPGKGVPGKGVPGKGVPGKG
VPGKGVPGKGVPGRGVPGKGVPGKGVPGKGVPGKGVPGKGVPGKGVPGKGVPGKGVPGKGVP
GKGVPGVGVPGKGVPGKGVPGKGVPGKGVPGKGVPGKGVPGKGVPGKGVPGKGVPGKGVPG
VGVPGKGVPGKGVPGKGVPGKGVPGKGVPGKGVPGKGVPGKGVPGKGVPGKGVPGLGVPGKG
VPGKGVPGKGVPGKGVPGKGVPGKGVPGKGVPGKGVPGKGVPGKGVPGVGVPGKGVPGKGVP
GKGVPGKGVPGKGVPGKGVPGKGVPGKGVPGKGVPGKGVPGVGVPGKGVPGKGVPGKGVPGK
GVPGKGVPGKGVPGKGVPGKGVPGKGVPGKGVPGIGVPGKGVPGKGVPGKGVPGKGVPGKGV
PGKGVPGKGVPGKGVPGKGVPGKGVPGVGVPGKGVPGKGVPGKGVPGKGVPGKGVPGKGVPGKGVPG
 
KGVPGKGVPGKGVPGVGVPGKGVPGKGVPGKGVPGKGVPGKGVPGKGVPGKGVPGKG
VPGKGVPGAGVPGKGVPGKGVPGKGVPGKGVPGKGVPGKGVPGKGVPGKGVPGKG;
 herein, the underlined part is repeating unit [(VPGKG)₉], and the bold-type part is the spacer (VPGXG);
SEQ ID NO:5 
VPGKGVPGKGVPGKGVPGKGVPGKGVPGKGVPGKGVPGKGVPGKGVPGVGVPGKGVP
GKGVPGKGVPGKGVPGKGVPGKGVPGKGVPGKGVPGKGVPGVGVPGKGVPGKGVPGK
GVPGKGVPGKGVPGKGVPGKGVPGKGVPGKGVPGKGVPGAGVPGKGVPGKGVPGKGVPGKGV
PGKGVPGKGVPGKGVPGKGVPGKGVPGKGVPGVGVPGKGVPGKGVPGKGVPGKGVPGKGVPGKGVPG
KGVPGKGVPGKGVPGKGVPGKGVPGVGVPGKGVPGKGVPGKGVPGKGVPGKGVPGKGVPGKGVPGKG
VPGKGVPGKGVPGLGVPGKGVPGKGVPGKGVPGKGVPGKGVPGKGVPGKGVPGKGVPGKGVP
GKGVPGVGVPGKGVPGKGVPGKGVPGKGVPGKGVPGKGVPGKGVPGKGVPGKGVPGKGVPG
VGVPGKGVPGKGVPGKGVPGKGVPGKGVPGKGVPGKGVPGKGVPGKGVPGKGVPGRGVPGK
GVPGKGVPGKGVPGKGVPGKGVPGKGVPGKGVPGKGVPGKGVPGKGVPGVGVPGKGVPGKGV
PGKGVPGKGVPGKGVPGKGVPGKGVPGKGVPGKGVPGKGVPGVGVPGKGVPGKGVPGKGVPG
KGVPGKGVPGKGVPGKGVPGKGVPGKGVPGKGVPGIGVPGKGVPGKGVPGKGVPGKGVPGKG
VPGKGVPGKGVPGKGVPGKGVPGKGVPGVGVPGKGVPGKGVPGKGVPGKGVPGKGVPGKGVPGKGVP
GKGVPGKGVPGKGVPGVGVPGKGVPGKGVPGKGVPGKGVPGKGVPGKGVPGKGVPGKGVPGK
GVPGKGVPGAGVPGKGVPGKGVPGKGVPGKGVPGKGVPGKGVPGKGVPGKGVPGKGVPGKGV
PGVGVPGKGVPGKGVPGKGVPGKGVPGKGVPGKGVPGKGVPGKGVPGKGVPGKGVPGVGVPG
KGVPGKGVPGKGVPGKGVPGKGVPGKGVPGKGVPGKGVPGKGVPGKG; herein, the underlined
 part is repeating unit [(VPGKG)₉], and the bold-type part is the spacer (VPGXG).

In a specific implementation scheme, the elastin-like protein rich in the amino functional group exists independently in the form of aqueous solution, and the compound containing succinimide group exists in the form of solid powder. In use, the compound containing the succinimide group is dissolved in ultrapure water, and then mixed with the elastin-like protein aqueous solution, and gelatinization occurs, to form an in-situ adhesion layer.

As a preferred scheme, the mass volume concentration of the aqueous solution of the elastin-like protein rich in the amino functional group is 50~650 mg/mL, preferably between 200~400 mg/mL, and more preferably 200 mg/mL;
as a preferred scheme, in use, the mass volume concentration of the aqueous solution of the compound containing the succinimide group is 0.5~1.5 g/mL, preferably 0.5~1.0 g/mL, and most preferably 0.75 g/mL;
as a preferred scheme, in use, the aqueous solution of the compound containing the succinimide group and the aqueous solution of the elastin-like protein rich in the amino functional group are mixed in a volume ratio of 1:(0.5~2.0), preferably 1:1; and
as a preferred scheme, after two types of the solution are mixed, the gelatinization time is 0~120 s. In the second aspect, the present application provides a preparation method for the two-component in-situ adhesive as described in the above first aspect, and comprising the following steps:
   1) the elastin-like protein rich in the amino functional group is mixed with water, to prepare aqueous solution of the elastin-like protein rich in the amino functional group;
   2) the compound containing the succinimide group is dissolved in water, to prepare aqueous solution of the compound containing the succinimide group;
and optionally, 3) the aqueous solution of the elastin-like protein rich in the amino functional group prepared in the Step 1) and the aqueous solution of the compound containing the succinimide group prepared in the Step 2) are separately packaged into a two-component injector. As a preferred scheme, the mass volume concentration of the aqueous solution of the elastin-like protein rich in the amino functional group prepared in the Step 1) is 50~650 mg/mL, preferably between 200~400 mg/mL, and more preferably 200 mg/mL;
as a preferred scheme, the mass volume concentration of the aqueous solution of the compound containing the succinimide group prepared in the Step 2) is 0.5~1.5 g/mL, preferably 0.5~1.0 g/mL, and most preferably 0.75 g/mL.

In the third aspect, the present application provides the use of the two-component in-situ adhesive as described in the above first aspect for preparation of a medicament for hemostasis, restoration, and/or regeneration of tissue or organ wounds;
preferably, the tissue is at least one selected from a meniscus tissue, a muscle tissue, a tendon tissue, a bone tissue, a connective tissue, an epidermal tissue, or a mucosal tissue;
preferably, the organ is at least one selected from skin, heart, liver, kidney, brain, or blood vessel;
preferably, the restoration is at least one selected from promoting closure, cell adhesion, proliferation, or differentiation of a damaged site;
further preferably, the medicament is used for assisting hemostasis in vascular reconstruction closure or assisting suture in cranial suture site; and
further preferably, the medicament is used for epidermal adhesion or intracranial adhesion.

### Beneficial Effect

The two-component in-situ adhesive based on the supercharged protein provided in the present application may be in-situ coated on the surface of tissue wounds, it utilizes a normal temperature ethyldimethylaminopropyl carbodiimide and N-hydroxysuccinimide (EDC-NHS) reaction between the amino group on the elastin-like protein rich in the amino functional group and the active succinimide of the compound containing the succinimide group, and at the same time, the amino group on the biological tissue protein also generates the EDC-NHS reaction (referring to the following Reaction Formula I) rapidly with the active succinimide, the in-situ and rapid gelatinization at the tissue wound is achieved, the excellent adhesion strength between the tissues is shown, and the excellent adhesion performance is also shown even on the moist tissue surface;

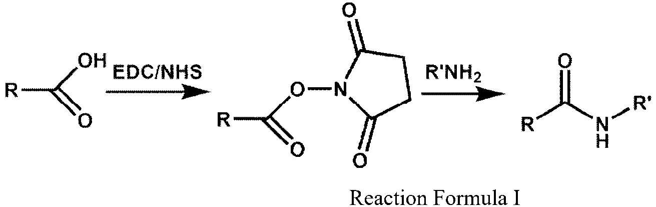

After the gelatinization, its adhesive surface is soft, and may be used to achieve the effects of moist wound hemostasis, wound restoration and the like. For example, it may be used for assisting hemostasis in vascular reconstruction closure, or assisting suture in cranial suture site; at the same time, the two-component in-situ adhesive of the present application also has the advantages of simple and rapid preparation process, no need for special devices, rapid adhesion and biocompatibility and the like. It may be used as a new generation of a portable instantaneous biomedical adhesive, and it has great application prospects in situations such as acute bleeding of wounds and complex wound surface treatment.

### Brief Description of the Drawings

One or more embodiments are exemplarily described by pictures in drawings corresponding to it, and these exemplary descriptions do not constitute limitations on the embodiments. Here, the specialized word "exemplary" means "used as an example, embodiment, or description". Here, any embodiments used as the "exemplary" description need not be interpreted as superior to or better than other embodiments.

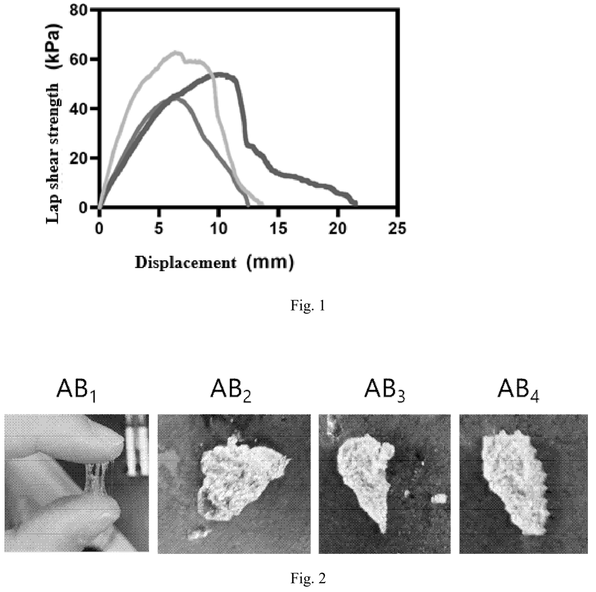

Fig. 1 shows the adhesion mechanical curve of a two-component in-situ adhesive prepared in Embodiment 1 on defatted pigskin; herein, the horizontal coordinate displays the adhesive tensile length (namely, displacement, and the unit is mm), and the vertical coordinate displays a lap shear strength (the unit is kPa);
Fig. 2 shows a cured morphology of a formed two-component adhesive based on SS-PEG-SS containing PEG of different molecular weights prepared in Embodiment 2 and an elastin-like protein as shown in SEQ ID NO: 3; herein, AB₁ is a two-component in-situ adhesive based on the elastin-like protein as shown in SEQ ID NO: 3 and SS-PEG-SS (herein the average relative molecular weight of PEG is 1000), AB₂ is a two-component in-situ adhesive based on the elastin-like protein as shown in SEQ ID NO: 3 and SS-PEG-SS (herein the average relative molecular weight of PEG is 2000), AB₃ is a two-component in-situ adhesive based on the elastin-like protein as shown in SEQ ID NO: 3 and SS-PEG-SS (herein the average relative molecular weight of PEG is 5000), and AB₄ is a two-component in-situ adhesive based on the elastin-like protein as shown in SEQ ID NO: 3 and SS-PEG-SS (herein the average relative molecular weight of PEG is 10000).

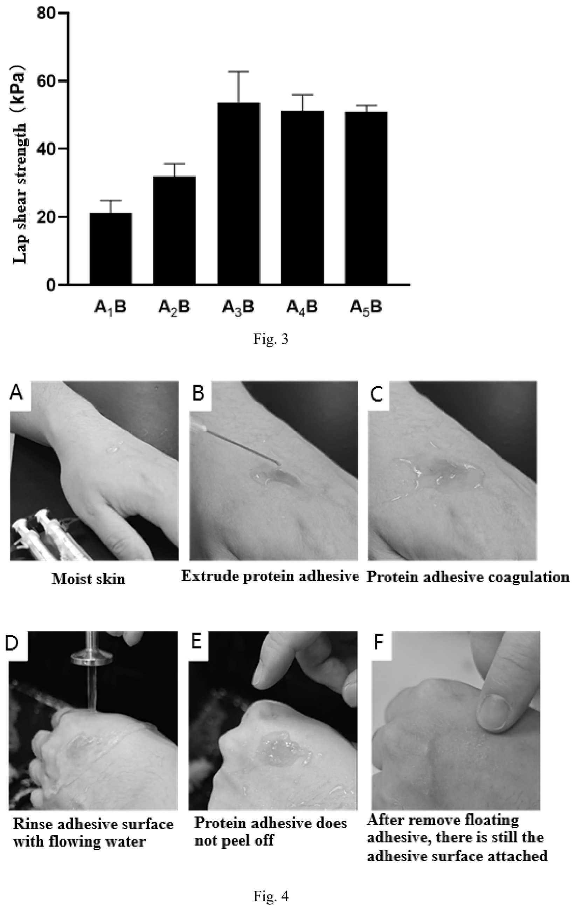

Fig. 3 shows the adhesion strengths of five two-component in-situ adhesives prepared in Embodiment 1 and Embodiment 3 on the defatted pigskin; herein, the horizontal coordinate displays the types of the two-component in-situ adhesives, herein, A₁B is a two-component in-situ adhesive based on the elastin-like protein as shown in SEQ ID NO: 1 and SS-PEG-SS (herein the average relative molecular weight of PEG is 5000), A₂B is a two-component in-situ adhesive based on the elastin-like protein as shown in SEQ ID NO: 2 and SS-PEG-SS (herein the average relative molecular weight of PEG is 5000), A₃B is a two-component in-situ adhesive based on the elastin-like protein as shown in SEQ ID NO: 3 and SS-PEG-SS (herein the average relative molecular weight of PEG is 5000), A₄B is a two-component in-situ adhesive based on the elastin-like protein as shown in SEQ ID NO: 4 and SS-PEG-SS (herein the average relative molecular weight of PEG is 5000), and A₅B is a two-component in-situ adhesive based on the elastin-like protein as shown in SEQ ID NO: 5 and SS-PEG-SS (herein the average relative molecular weight of PEG is 5000); and the vertical coordinate displays the lap shear strength (the unit is kPa).
Fig. 4 shows that the two-component protein adhesive (Fig. B) is extruded on the moist skin (Fig. A), and the adhesive is visibly coagulated after about 20 s (Fig. C); subsequently, the tissue interface with the adhesive is rinsed with flowing water for 1 min (Fig. D), and it is visible that: the protein adhesive is not detached (Fig. E); then, the adhesive surface is vigorously rubbed with fingers, and there are still some protein adhesives attached to the tissue surface (Fig. F).

### Detailed Description of the Invention

In order to make purposes, technical schemes, and advantages of the present application clearer, the technical schemes in the embodiments of the present application are clearly and completely described below. Apparently, the embodiments described are a part of the embodiments of the present application, not all of the embodiments. Based on the embodiments in the present application, all other embodiments obtained by those of ordinary skill in the art without creative labor shall fall within the scope of protection of the present application.

Unless otherwise expressly stated, the term "including" or its variations such as "containing" or "comprising" throughout the description and claims shall be understood to include elements or components stated, and shall not exclude other elements or components.

In addition, in order to describe present application better, numerous specific details are provided in the following specific implementation modes. It should be understood by those skilled in the art that without certain specific details, the present application may still be implemented. In some embodiments, raw materials, elements, methods, means and the like that are well-known to those skilled in the art are not described in detail, as to highlight the subject matter of the present application.

Below, the present application is described in detail.

On the one hand, the present application provides a two-component in-situ adhesive based on a supercharged protein, comprising an elastin-like protein rich in an amino functional group and a compound containing succinimide group.

As a preferred scheme, the elastin-like protein rich in the amino functional group is an engineered expression protein with (VPGKG)₉ as a basic repeating unit, with each pair of repeating units separated by (VPGXG), the number n of the repeating units n is between 3~18, preferably between 8~18, and X in the spacer (VPGXG) is any natural amino acids except proline.

In a preferred specific implementation scheme, X in the spacer (VPGXG) is valine, alanine, leucine, isoleucine, or arginine.

In a preferred specific implementation scheme, the elastin-like protein rich in the amino functional group contains an amino acid sequence selected from or composed of the following: SEQ ID NO: 1, SEQ ID NO: 2, SEQ ID NO: 3, SEQ ID NO: 4, and SEQ ID NO: 5.

As a preferred scheme, the compound containing the succinimide group is any one or more selected from the following: bis-succinimide succinate polyethylene glycol (SS-PEG-SS), succinimide acetate, and disulfide bis-succinimide propionate, preferably SS-PEG-SS, and further preferably, the average relative molecular weight of PEG in the SS-PEG-SS is between 2000-10000, preferably between 5000-10000.

On the other hand, the present application provides a preparation method for the above two-component in-situ adhesive based on the supercharged protein, comprising the following steps:
1) the elastin-like protein rich in the amino functional group is mixed with water, to prepare aqueous solution of the elastin-like protein rich in the amino functional group; preferably, the mass volume concentration of the aqueous solution of the elastin-like protein rich in the amino functional group is 50~650 mg/mL, preferably between 200~400 mg/mL, and more preferably 200 mg/mL;
2) the compound containing succinimide group is dissolved in water, to prepare aqueous solution of the compound containing the succinimide group; preferably, the mass volume concentration of the aqueous solution of the compound containing the succinimide group is 0.5~1.5 g/mL, preferably 0.5~1.0 g/mL, and more preferably 0.75 g/mL;
and optionally, 3) the aqueous solution of the elastin-like protein rich in the amino functional group prepared in the Step 1) and the aqueous solution of the compound containing the succinimide group prepared in the Step 2) are separately packaged into a two-component injector. In addition, the present application provides the use of the above two-component in-situ adhesive for preparation of a medicament for hemostasis, restoration, and/or regeneration of tissue wound. For example, the medicament is used for assisting hemostasis in vascular reconstruction closure or assisting suture in cranial suture site.

The present application is further described below by the embodiments; in the following embodiments, unless otherwise specified, biological and chemical materials used are all commercially available products; and the elastin-like protein used may be prepared by any suitable technologies known to those skilled in the art.

In the following embodiments, the used elastin-like proteins rich in the amino functional group are all synthesized by Beijing Lanthanide Biotechnology Co., Ltd; the used SS-PEG-SS containing PEG with different molecular weights is all purchased from Xi'an Kaixin Biotechnology Co., Ltd; the used biological tissues are all obtained by the inventor through a conventional separation method from commercially purchased experimental animals; and the used pig casing (Trade Name: "Pixiaojian Natural Pig Casing", purchased from Shenzhen Pinyuepin Food Co., Ltd.) is obtained by the inventor through a commercial purchase method.

### Embodiment 1

1) Adding 200 mg of the elastin-like protein rich in the amino functional group to 1 mL of ultrapure water, and mixed evenly, to obtain 200 mg/mL of homogeneous protein solution, denoted as component solution A (i.e., elastin-like protein solution); the elastin-like protein had an amino acid sequence as shown in SEQ ID NO: 3;
2) Weighing 1 g of SS-PEG-SS powder (herein the average relative molecular weight of PEG was 5000), adding it to 1 mL of ultrapure water, and mixed evenly, to obtain component B solution (i.e., SS-PEG-SS solution);
3) Separately encapsulate component solution A and the component solution B into a two-component injector, to obtain the two-component in-situ adhesive A₃B in the present application.

When using, the component solution A and B was successively coated on the surface of pigskin, pork, pig liver and pig heart soft tissue samples, and after being mixed evenly, it was quickly lapped to the adhesive surface with the same type of a substrate plate, and it was gelated for 2 s, to form an in-situ adhesion layer; herein, the volume ratio of the elastin-like protein solution to SS-PEG-SS solution was 1:1.

Using the SHIMADZU SLBL-500N tensile machine, the adhesive performance of the adhesive was evaluated by using the shear adhesion strength, as to represent the adhesion strength of the adhesive on the defatted pigskin, pork, pig liver, and pig heart soft tissue samples.

Fig. 1 was an adhesion mechanical curve of the adhesive prepared in this embodiment on the defatted pigskin soft tissue sample, herein, 3 curves were respectively from 3 parallel experiments, and its preparation and testing processes were consistent. Fig. 1 showed that: the adhesion strength obtained from the testing of the two-component in-situ adhesive of the present application based on the elastin-like protein as shown in SEQ ID NO: 3 and SS-PEG-SS was 53.54±7.47 kPa; and this result indicated that: the two-component in-situ adhesive of the present application based on the elastin-like protein as shown in SEQ ID NO: 3 and SS-PEG-SS had good adhesion performance on biological epidermal tissues.

### Embodiment 2

In this embodiment, three types of two-component in-situ adhesives of the present application: AB₁, AB₂, AB₃, and AB₄, were prepared through the following steps, herein:
AB₁ was a two-component in-situ adhesive based on the elastin-like protein as shown in SEQ ID NO: 3 and SS-PEG-SS solution (herein the average relative molecular weight of PEG was 1000);
AB₂ was a two-component in-situ adhesive based on the elastin-like protein as shown in SEQ ID NO: 3 and SS-PEG-SS solution (herein the average relative molecular weight of PEG was 2000);
AB₃ was a two-component in-situ adhesive based on the elastin-like protein as shown in SEQ ID NO: 3 and SS-PEG-SS solution (herein the average relative molecular weight of PEG was 5000); and
AB₄ was a two-component in-situ adhesive based on the elastin-like protein as shown in SEQ ID NO: 3 and SS-PEG-SS solution (herein the average relative molecular weight of PEG was 10000); The preparation method was as follows: add 200 mg of the elastin-like protein with the amino acid sequence as shown in SEQ ID NO: 3 to 1 mL of ultrapure water, and mixed evenly, to obtain 200 mg/mL of homogeneous protein solution component A solution; SS-PEG-SS solution containing PEG with different molecular weights was prepared into 750 mg/mL of aqueous solution, and it was prepared into component B solution. The component solution A and B was filled into two thrusters of a two-component injector, to form a pre-filled two-component adhesive.

When using, the component solution A and B was injected in-situ in a volume ratio of 1:1, to form the two-component adhesive.

Observe the curing time and morphology of AB₁, AB₂, AB₃, and AB₄ during the gelatinization process (see Fig. 2), and the rupture strength was detected (see Table 1).

Detection method for rupture strength: after a pig casing was appropriately treated (including: the casing was rinsed to remove excess salt and it was soaked in water to maintain the elasticity), it was installed on a rupture fixture, and detected for good sealing, a hole with a diameter of 0.5 mm was drilled at the center of the fixture, to simulate a tissue leakage point. The two-component adhesives AB₁, AB₂, AB₃, and AB₄ were injected onto a casing membrane of the rupture fixture, a rupture site was closed, and the total volume of injection was 1 mL. After the injection was completed, timing started until the two-component instantaneous adhesive was completely cured, and the time used was calculated as the curing time of the two-component instantaneous adhesive. The air was injected into the sealed fixture, and the gas was transmitted into the interior of the fixture through a pressure gauge. When the cured adhesive attached to the casing might not withstand the increased air pressure, the gas might leak from the perforation on the casing, and the pressure gauge reading might be instantly decreased; and the highest reading on the pressure gauge was recorded as the rupture strength of the two-component instantaneous in-situ adhesive for this ratio.

**Table 1: Morphology, curing time, and rupture strength of adhesive formed by SS-PEG-SS solution containing PEG with different molecular weights and elastin-like protein:**

| | AB₁ | AB₂ | AB₃ | AB₄ |
|---|---|---|---|---|
| Curing time | Not curing | About 5 min | About 90 s | About 30 s |
| Rupture strength | 20 kPa | 5 kPa | 30 kPa | 35 kPa |
| Morphology | Forming liquid adhesive after 0.5 h | Solid gel-like, elastic, and very soft | Solid gel-like, elastic, and soft | Solid gel-like, elastic, and relatively soft |

Fig. 2 showed the morphology of the SS-PEG-SS adhesive containing PEG with different molecular weights prepared in this embodiment after complete gelatinization and peeling off from the rupture fixture. The result in Fig. 2 showed that SS-PEG-SS containing PEG with different molecular weights had different effects on the rupture strength of the adhesive: the molecular weight of PEG was higher, the curing speed was faster, the rupture strength was stronger, and the adhesive body was harder. It was considered that when the two-component adhesive was applied *in vivo,* the higher adhesive hardness might increase discomfort, it was not necessarily better for the two-component adhesive to have the stronger rupture strength or the harder adhesive body; at the same time, when the two-component adhesive was applied *in vivo,* the suitable curing time (for example, 5 s~2 min) was necessary, thus it was convenient for doctors to operate, and the inability to quickly remove the adhesive after incorrect application was avoided.

The above considerations and results were balanced: the adhesive of the present application prepared by SS-PEG-SS in which the average relative molecular weight of PEG was 2000, 5000, and 10000 (especially the latter two) had the better comprehensive performance on moist biological tissues, and different combinations might be selected according to usage routes and sites of action.

### Embodiment 3

In this embodiment, four types of two-component in-situ adhesives of the present application: A₁B, A₂B, A₄B, and A₅B, were prepared through the following steps, herein, A₁B was a two-component in-situ adhesive based on the elastin-like protein as shown in SEQ ID NO: 1 and SS-PEG-SS (herein the average relative molecular weight of PEG was 5000), A₂B was a two-component in-situ adhesive based on the elastin-like protein as shown in SEQ ID NO: 2 and SS-PEG-SS (herein the average relative molecular weight of PEG was 5000), A₄B was a two-component in-situ adhesive based on the elastin-like protein as shown in SEQ ID NO: 4 and SS-PEG-SS (herein the average relative molecular weight of PEG was 5000), and A₅B was a two-component in-situ adhesive based on the elastin-like protein as shown in SEQ ID NO: 5 and SS-PEG-SS (herein the average relative molecular weight of PEG was 5000):
1) Adding 200 mg of each of the four elastin-like proteins rich in the amino functional group as shown in SEQ ID NO: 1, SEQ ID NO: 2, SEQ ID NO: 4, and SEQ ID NO: 5 into 1 mL of ultrapure water, and mixed evenly, to obtain 200 mg/mL of homogeneous protein solution, it was marked as component solution A₁, A₂, A₄, and As respectively;
2) Weighing 1 g of SS-PEG-SS powder, adding it to 1 mL of ultrapure water, and mixed evenly, to obtain component solution B;
3) Separately encapsulate the component solution A₁, or the component solution A₂, or the component solution A₄, or the component solution A₅, and the component solution B into a two-component injector, to obtain the two-component instantaneous in-situ adhesives A₁B, A₂B, A₄B, and A₅B of the present application.

When using, the component solution A₁ and B or A₂ and B or A₄ and B or As and B were successively coated on the surface of a defatted pigskin soft tissue sample, and after being mixed evenly, it was quickly lapped to the adhesive surface by using the same type of a substrate plate, and gelated for 2 s, to form an in-situ adhesive layer; herein, the volume ratio of the elastin-like protein solution rich in the amino functional group to the SS-PEG-SS solution was 1:1.

The four types of the two-component instantaneous in-situ adhesives A₁B, A₂B, A₄B, and A₅B prepared in this embodiment were lapped and bonded on the defatted pigskin tissue, and subjected to a tensile test (the method was the same as Embodiment 1). Its result and the test result of the two-component instantaneous in-situ adhesive A₃B prepared in Embodiment 1 were shown in Fig. 3. Figure 3 showed that: the tested adhesion strength of the two-component in-situ adhesive A₁B of the present application based on the elastin-like protein as shown in SEQ ID NO: 1 and SS-PEG-SS (herein the average relative molecular weight of PEG was 5000) was 21.24±2.97 kPa, the tested adhesion strength of the two-component in-situ adhesive A₂B of the present application based on the elastin-like protein as shown in SEQ ID NO: 2 and SS-PEG-SS (herein the average relative molecular weight of PEG was 5000) was 31.91±3.05 kPa, the tested adhesion strength of the two-component in-situ adhesive A₃B of the present application based on the elastin-like protein as shown in SEQ ID NO: 3 and SS-PEG-SS (herein the average relative molecular weight of PEG was 5000) was 53.54±7.47 kPa (namely, the adhesion strength tested in Embodiment 1), the tested adhesion strength of the two-component in-situ adhesive A₄B of the present application based on the elastin-like protein as shown in SEQ ID NO: 4 and SS-PEG-SS (herein the average relative molecular weight of PEG was 5000) was 51.49±3.86 kPa, and the tested adhesion strength of the two-component in-situ adhesive A₅B of the present application based on the elastin-like protein as shown in SEQ ID NO: 5 and SS-PEG-SS (herein the average relative molecular weight of PEG was 5000) was 50.22±1.81 kPa.

The above results indicated that: the two-component in-situ adhesives of the present application based on the elastin-like protein as shown in SEQ ID NO: 1 or SEQ ID NO: 2 or SEQ ID NO: 3 or SEQ ID NO: 4 or SEQ ID NO: 5 and SS-PEG-SS had good adhesion performance on the moist biological tissues, might meet the tissue adhesion needs in clinical practice, and might provide diversified solution schemes for different tissue adhesion requirements. Herein, the adhesion performance of the two-component in-situ adhesive based on the elastin-like protein as shown in SEQ ID NO: 3 or SEQ ID NO: 4 or SEQ ID NO: 5 and SS-PEG-SS was relatively high, especially the adhesion performance of the two-component in-situ adhesive A₃B based on the elastin-like protein as shown in SEQ ID NO: 3 and SS-PEG-SS was the best; when the sequence length was greater than the sequence shown in SEQ ID NO: 3, the adhesion strength might not increase rapidly (A₃B, A₄B, and A₅B had the similar adhesion strength), but the protein expression efficiency was decreased. Therefore, the sequence SEQ ID NO: 3 was an optimal elastin-like protein sequence.

### Embodiment 4

In this embodiment, the adhesion effect of the two-component in-situ adhesive AB₄ prepared in Embodiment 2 on the moist skin was tested through the following steps:
The two-component protein adhesive (Fig. B in Fig. 4) was extruded on the moist skin (Fig. A in Fig. 4), and after about 20 s, the adhesive body was visibly coagulated (Fig. C in Fig. 4); subsequently, the tissue interface with the adhesive was rinsed with flowing water for 1 min (Fig. D in Fig. 4), and it was visible that: the protein adhesive was not detached (Fig. E in Fig. 4); then, the adhesive surface was vigorously rubbed with fingers, and there were still some protein adhesives attached to the tissue surface (Fig. F in Fig. 4).

It is indicated from the above embodiments that the two-component instantaneous in-situ adhesive of the present application may achieve the in-situ and rapid gelatinization on the surface of moist biological tissues, shows the excellent adhesion performance, and may be used to achieve the effects of moist wound hemostasis, wound restoration and the like. For example, it may be used for assisting hemostasis in vascular reconstruction closure, or assisting suture in cranial suture site. Finally, it should be noted that the above embodiments are only used to describe technical schemes of the present application, and not to limit it; although the present application is already described in detail with reference to the aforementioned embodiments, it should be understood by those skilled in the art that: the technical schemes recorded in the aforementioned embodiments may still be modified, or some of technical features in the technical schemes are equivalently replaced; and these modifications or replacements do not make the essence of the corresponding technical schemes depart from the spirit and scope of the technical schemes in each embodiment of the present application.

### Industrial Applicability

The two-component in-situ adhesive based on the supercharged protein provided in the present application may be used for in-situ and rapid gelatinization at tissue wounds, showing excellent adhesion strength between tissues, and also showing excellent adhesion performance even on the moist tissue surface; after the gelatinization, its adhesive surface is soft, and may be used to achieve the effects of moist wound hemostasis, wound restoration and the like; at the same time, the adhesive of the present application also has the advantages of simple and rapid preparation process, no need for special devices, rapid adhesion and biocompatibility and the like. It may be used as a new generation of a portable nstantaneous biomedical adhesive, and it has great application prospects in situations such as acute bleeding of wounds and complex wound surface treatment.

## Claims

1. A two-component in-situ adhesive based on a supercharged protein, comprising:
(1) an elastin-like protein rich in an amino functional group, and
(2) a compound containing a succinimide group.

2. The two-component in-situ adhesive according to claim 1, wherein the elastin-like protein rich in the amino functional group contains n [(VPGKG)₉] repeating units, with each pair of [(VPGKG)₉] repeating units separated by a spacer (VPGXG); wherein, n is an integer between 3~18, and X is any natural amino acids except proline;
and/or, the compound containing the succinimide group is any one or more selected from the following: bis-succinimide succinate polyethylene glycol, succinimide acetate, and disulfide bis-succinimide propionate.

3. The two-component in-situ adhesive according to claim 2, wherein in the elastin-like protein rich in the amino functional group, the number n of the repeating units [(VPGKG)₉] is an integer between 8~18, and/or, in the spacer (VPGXG), X is valine, arginine, leucine, isoleucine, or alanine;
and/or, the compound containing the succinimide group is bis-succinimide succinate polyethylene glycol.

4. The two-component in-situ adhesive according to claim 3, wherein the elastin-like protein rich in the amino functional group contains an amino acid sequence selected from or composed of the following: SEQ ID NO: 1, SEQ ID NO: 2, SEQ ID NO: 3, SEQ ID NO: 4, and SEQ ID NO: 5; and/or, the compound containing the succinimide group is bis-succinimide succinate polyethylene glycol, wherein the average relative molecular weight of polyethylene glycol is between 2000-10000.

5. The two-component in-situ adhesive according to claim 4, wherein the elastin-like protein rich in the amino functional group contains an amino acid sequence selected from or composed of the following: SEQ ID NO: 3, SEQ ID NO: 4, and SEQ ID NO: 5;
and/or, the compound containing the succinimide group is bis-succinimide succinate polyethylene glycol, wherein the average relative molecular weight of polyethylene glycol is between 5000-10000.

6. The two-component in-situ adhesive according to any one of claims 1~5, wherein the elastin-like protein rich in the amino functional group exists independently in the form of aqueous solution, and the compound containing the succinimide group exists in the form of solid powder; in use, the compound containing the succinimide group is dissolved with ultrapure water, and then mixed with the elastin-like protein aqueous solution, and gelatinization occurs, to form an in-situ adhesion layer.

7. The two-component in-situ adhesive according to claim 6, wherein the mass volume concentration of the aqueous solution of the elastin-like protein rich in the amino functional group is 50~650 mg/mL;
and/or, in use, the compound containing the succinimide group is prepared into aqueous solution with a mass volume concentration of 0.5~1.5 g/mL;
and/or, in use, the aqueous solution of the compound containing the succinimide group and the aqueous solution of the elastin-like protein rich in the amino functional group are mixed in a volume ratio of 1:(0.5~2.0);
and/or, the gelatinization time is 0~120 s.

8. The two-component in-situ adhesive according to claim 7, wherein the mass volume concentration of the aqueous solution of the elastin-like protein rich in the amino functional group is between 200~400 mg/mL;
and/or, the mass volume concentration of the aqueous solution of the compound containing the succinimide group is 0.5~1.0 g/mL;
and/or, in use, the aqueous solution of the compound containing the succinimide group and the aqueous solution of the elastin-like protein rich in the amino functional group are mixed in a volume ratio of 1:1.

9. A preparation method for the two-component in-situ adhesive according to any one of claims 1~8, comprising the following steps:
1) mixing the elastin-like protein rich in the amino functional group with water, to prepare aqueous solution of the elastin-like protein rich in the amino functional group;
2) dissolving the compound containing the succinimide group in water, to prepare aqueous solution of the compound containing the succinimide group;
and optionally, 3) packaging the aqueous solution of the elastin-like protein rich in the amino functional group prepared in the Step 1) and the aqueous solution of the compound containing the succinimide group prepared in the Step 2) separately into a two-component injector.

10. The preparation method according to claim 9, wherein the mass volume concentration of the aqueous solution of the elastin-like protein rich in the amino functional group prepared in the Step 1) is 50~650 mg/mL;
and/or, the mass volume concentration of the aqueous solution of the compound containing the succinimide group prepared in the Step 2) is 0.5~1.5 g/mL.

11. The preparation method according to claim 10, wherein the mass volume concentration of the aqueous solution of the elastin-like protein rich in the amino functional group prepared in the Step 1) is 200~400 mg/mL;
and/or, the mass volume concentration of the aqueous solution of the compound containing the succinimide group prepared in the Step 2) is 0.5~1.0 g/mL.

12. The use of the two-component in-situ adhesive according to any one of claims 1~8 for preparation of a medicament for hemostasis, restoration, and/or regeneration of a tissue or organ wound.

13. The use according to cla im 12, wherein the tissue is at least one selected from a meniscus tissue, a muscle tissue, a tendon tissue, a bone tissue, a connective tissue, an epidermal tissue, or a mucosal tissue;
and/or, the organ is at least one selected from skin, heart, liver, kidney, brain, or blood vessel;
and/or, the restoration is at least one selected from promoting closure, cell adhesion, proliferation, or differentiation of a damaged site.
